Europäisches Patentamt

European Patent Office

Office européen des brevets

(19)

(11) Veröffentlichungsnummer : **0 041 109**
**B1**

(12) **EUROPÄISCHE PATENTSCHRIFT**

(45) Veröffentlichungstag der Patentschrift :
15.12.82

(21) Anmeldenummer : **81102267.2**

(22) Anmeldetag : **26.03.81**

(51) Int. Cl.³ : **C 07 C 53/08, C 07 C 51/16**

(54) **Verfahren zur Herstellung von Essigsäure aus sek.-Butylacetat.**

(30) Priorität : **24.05.80 DE 3019932**

(43) Veröffentlichungstag der Anmeldung :
**09.12.81 (Patentblatt 81/49)**

(45) Bekanntmachung des Hinweises auf die Patenterteilung : **15.12.82 Patentblatt 82/50**

(84) Benannte Vertragsstaaten :
**FR GB IT NL**

(56) Entgegenhaltungen :
**US A 3 505 400**

(73) Patentinhaber : **CHEMISCHE WERKE HÜLS AG**
**Postfach 1320**
**D-4370 Marl 1 (DE)**

(72) Erfinder : **Brockhaus, Rudolf, Dr.**
**Holsteiner Strasse 19**
**D-4370 Marl (DE)**
Erfinder : **Rademacher, Hans, Dr.**
**Lipper Weg 195**
**D-4370 Marl (DE)**
Erfinder : **Scholz, Bernhard, Dr.**
**Zu den Mühlen 10**
**D-4370 Marl (DE)**

## Verfahren zur Herstellung von Essigsäure aus sek.-Butylacetat

Zur Herstellung von Essigsäure wendet man mehrere Verfahren technisch an. Die bekanntesten Verfahren sind die Oxidation von Acetaldehyd in flüssiger Phase, die Oxidation von n-Butan oder Leichtbenzin in flüssiger Phase, die katalytische Oxidation von n-Butenen in der Gasphase sowie die Synthese aus Methanol und Kohlenmonoxid.

Es ist weiterhin bekannt, Essigsäure durch Oxidation von sek.-Butylacetat in flüssiger Phase bei Temperaturen von 150 bis 250 °C und Drücken von 50 bis 150 bar herzustellen (DE-PS 12 69 610 = US-PS 3 362 987 und DE-PS 13 01 809 = US-PS 3 505 400). Sek.-Butylacetat erhält man durch Addition von Essigsäure an n-Butene. Bei der Oxidation von sek.-Butylacetat zu Essigsäure entstehen als Nebenprodukte leicht siedende Zwischenprodukte wie Acetaldehyd, Methylethylketon, Methyl- und Ethylacetat u. a. sowie Kohlenoxide und Ameisensäure. Die Zwischenprodukte lassen sich als Leichtsieder gut aus dem Reaktionsaustrag abtrennen, erneut in den Oxidationsprozeß einleiten und weitgehend in Essigsäure überführen. Mit der Leichtsiederrückführung kann man eine Essigsäureausbeute von 58,5 Gew.-% bezogen auf das in Form von sek.-Butylacetat eingesetzte Buten, erzielen (W. Krönig, The Proceedings of the 7. World Petroleum Congress, Vol. V. 559 bis 565 (1967)). Diese Ausbeute reicht jedoch für eine wirtschaftliche Nutzung der Verfahrensweise nicht aus.

Der größte Teil der Ausbeuteverluste ist auf die Bildung von $CO_2$ und CO (Produkte der Totaloxidation) zurückzuführen. Ein beträchtlicher Teil der in Form von sek.-Butylacetat zur Oxidation eingesetzten Butene (6,9 %) wird aber auch in Hochsieder umgewandelt. Zudem fällt bei diesem Verfahren eine relativ große Menge an Ameisensäure, bis zu 5, 7 Gew.-% an, deren Reingewinnung jedoch zu aufwendig ist, so daß sie vernichtet werden muß.

Nach diesem bekannten Verfahren führt man die Oxidation von sek.-Butylacetat zu Essigsäure vorteilhafterweise in Abwesenheit von Katalysatoren durch (DE-PS 12 69 610 = US-PS 3 362 987, Salte 1, Zeilen 47 bis 49 und DE-PS 13 01 809 = US-PS 3 505 400, Spalte 3, Zeilen 56 bis 58). Man kann bei dem Verfahren der DE-PSS 12 69 610 und 13 01 809 auch die bekannten Oxidationskatalysatoren, wie Kobaltacetat, Manganacetat und Chromacetat verwenden (DE-PS 12 69 610, Spalte 1, Zeilen 49 bis 51 und DE-PS 13 01 809, Spalte 3, Zeilen 58 bis 60). Diese Oxidationskatalysatoren setzt man bei Oxidationsreaktionen allgemein in Konzentrationen oberhalb 300 ppm, beispielsweise von 300 bis 100 000 ppm ein. (DE-AS 25 16 547 = US-PS 3 923 882 : 300 bis 30 000 ppm Co, DE-AS 25 13 678 = GB-PS 1 483 724 : 500 bis 5 000 ppm Co, DE-OS 26 32 898 = US-PSS 4 086 267 und 4 131 741 : 1 000 bis 100 000 ppm Co, DE-OS 28 16 835 : 500 bis 7 000 ppm Co).

Infolge geringerer Löslichkeit der Katalysatoren kommt es bei diesen Konzentrationen zu störenden Katalysatorablagerungen, beispielsweise von Kobaltverbindungen im Reaktor und in den Leitungen.

Die Abbildung 1 der Beispiele 3 bis 8 zeigt die ungünstige Wirkung der bisher üblichen höheren Kobalt-Konzentrationen und bestätigt damit die Lehre der DE-PS 12 69 610 (= US-PS 3 362 987) und der DE-PS 13 01 809 (= US-PS 3 505 400), daß man die Oxidation von sek.-Butylacetat zu Essigsäure statt bei diesen bisher üblichen Kobalt-Konzentrationen vorteilhafterweise in Abwesenheit von Katalysatoren durchführt.

Ein weiteres Problem stellt die Korrosion der Reaktionsapparate dar. Zwar wird in der DE-PS 12 94 361 (= US-PS 3 493 609) eine Maßnahme beschrieben, wie diese Korrosion verringert werden kann. Die Korrosion der dem Reaktionsmedium unter den dort beschriebenen Bedingungen ausgesetzten Metallwände ist jedoch noch immer so groß, daß die durch diese Korrosion verursachte Konzentration an Fremdmetallen wie Fe, Cr und Ni im Reaktionsgemisch 50 bis 100 ppm beträgt. Diese an sich sehr geringe Konzentration ist so hoch, daß dadurch der Einfluß eventuell zugesetzter Katalysator-Metallsalze völlig überspielt wird.

Die bekannten Verfahren der Oxidation von sek.-Butylacetat zu Essigsäure in flüssiger Phase haben somit erhebliche Nachteile, indem sie nur zu niedrigen Ausbeuten an Essigsäure führen und eine größere Menge unerwünschter Nebenprodukte wie Ameisensäure und Hochsieder liefern.

Damit stellt sich die Aufgabe, ein Verfahren zu finden, das bei der Oxidation von sek.-Butylacetat zu Essigsäure in flüssiger Phase in einfacher Weise zu höheren Ausbeuten an Essigsäure führt und die Bildung von Ameisensäure und Hochsiedern stark reduziert.

Diese Aufgabe wird erfindungsgemäß entsprechend den Angaben der Patentansprüche gelöst.

Überraschenderweise wurde nun gefunden, daß man die Selektivität der Flüssigphasenoxidation von sek.-Butylacetat zu Essigsäure wesentlich verbessern kann, wenn man als Katalysator ein Kobaltsalz in einer Konzentration von 1 bis 300 ppm zum Reaktionsmedium zufügt und durch Aufrechterhaltung eines Sauerstoff-Überschusses im gesamten Reaktionsraum einschließlich Kühler die Korrosion so weit zurückdrängt, daß die Konzentration an unerwünschten Metallionen (Fe, Ni, Cr) im Reaktionsmedium, die auf die Korrossion zurückzuführen ist, kleiner ist als die zugesetzte Kobaltkonzentration bzw. diese bei Kobaltkonzentrationen < 10 ppm nicht wesentlich übersteigt. Den Sauerstoffgehalt im gesamten Reaktionsraum einschließlich des Kühlers und der Rohrleitungen sowie im Abgas hält man auf 1 bis 6, vorzugsweise 3 bis 5 Volumenprozent. Dieses erreicht man durch folgende Maßnahmen : Man stimmt das Sauerstoff-Angebot und die Reaktionstemperatur so aufeinander ab, daß der Sauerstoffgehalt im gesamten Reaktionsraum einschließlich des Kühlers und der Rohrleitungen sowie in dem durch Kühlung von organischen Bestandteilen befreiten Abgas 1 bis 6, vorzugsweise 3 bis 5 Volumenprozent beträgt. Beispielsweise erhöht man bei einer konstanten Reaktionstemperatur das Sauerstoff-Angebot so lange, bis der gewünschte Sauerstoff-Gehalt im Abgas erreicht ist. Zusätzlich dosiert man soviel Sauerstoff bzw. Luft in den Kühler und/oder in die Rohrleitung zwischen Reaktionsbehälter und Kühler, daß auch an diesen Stellen der erforderliche Sauerstoffgehalt eingehalten wird. Diese zusätzliche Dosierung von Sauerstoff bzw. Luft ist erforderlich, da diese Stellen der Apparatur zum Teil von heißer Reaktionsflüssigkeit in Abwesenheit der noch Sauerstoff enthaltenden Reaktionsgase durchströmt werden.

Die anfallende Reaktionswärme führt man bevorzugt mittels Siedekühlung ab, wobei das im Kühler kondensierende Produkt relativ wenig Essigsäure und Ameisensäure enthält und bei relativ niedriger Temperatur anfällt. Diese bevorzugte Maßnahme vermindert zusätzlich die Korrosion.

Wie Abb. 1 der Beispiele 3 bis 8 zeigt, werden mit Co-Konzentrationen von 1 bis 300 ppm bessere Essigsäure-Selektivitäten als ohne Anwendung eines Katalysators erzielt. Vorzugsweise liegt die Kobalt-Konzentration bei 3 bis 100 ppm und insbesondere bei 10 bis 50 ppm. Bei diesen Kobalt-Konzentrationen erhält man optimale Essigsäure-Selektivitäten. Das Kobalt kann in Form eines beliebigen, im sauren Reaktionsmedium ausreichend löslichen Salzes zugegeben werden, bevorzugt wird Co-(II)-Acetat angewendet.

Bei Kobalt-Konzentrationen unter 1 ppm wird keine wesentliche Verbesserung der Essigsäureausbeute gegenüber dem Stand der Technik erreicht. Dasselbe ist der Fall bei Co-Konzentrationen über 300 ppm. Bei Co-Konzentrationen über 300 ppm nimmt die Selektivität stark ab. Zusätzlich treten bei sehr hohen Katalysatorkonzentrationen infolge geringer Löslichkeit des Katalysators im Reaktionsmedium Ablagerungen im Reaktor und in Produktleitungen auf.

Die erforderliche Reaktionstemperatur beträgt 150 bis 190 °C, vorzugsweise 160 bis 180 °C und liegt damit um ca. 20 bis 30 K unter den in den Beispielen der DE-PSS 12 69 610 (= US-PS 3 362 987) und 13 01 809 (= US-PS 3 505 400) genannten Temperaturen. Bei Reaktionstemperaturen unter 150 °C ist die Reaktionsgeschwindigkeit sehr gering und wirtschaftlich uninteressant. Bei Temperaturen über 190 °C sinkt die Essigsäureausbeute sehr stark ab und es kann keine wesentliche Verbesserung mehr gegenüber dem Stand der Technik erreicht werden.

Die Oxidation erfolgt bei Drücken von 7 bis 30 bar, insbesondere bei 10 bis 20 bar mit Sauerstoff oder sauerstoffhaltigen Gasen wie Luft. Der Druck ist zur Aufrechterhaltung der flüssigen Phase erforderlich. Dies ist eine wesentliche Vereinfachung gegenüber den bekannten Verfahren, bei denen Drücke von 50 bis 150 bar erforderlich sind und ermöglicht eine erhebliche Erniedrigung des Kapital-Investments.

Beim erfindungsgemäßen Arbeiten erreicht man eine Essigsäureausbeute bis 75 % bezogen auf die in Form von sek.-Butylacetat zur Oxidation eingesetzten Butene. Dies bedeutet gegenüber der bekannten Verfahrensweise eine Ausbeutesteigerung von nahezu 30 %. Ein besonderer Vorteil des erfindungsgemäßen Verfahrens ist der unerwartet niedrige Anfall an unerwünschter Ameisensäure von 1,5 bis 3,8 %. Auch der Anfall an Hochsiedern ist wesentlich verringert.

Das nicht umgesetzte sek.-Butylacetat und Zwischenprodukte der Oxidation führt man vorzugsweise in den Oxidator zurück.

Die erhaltene Essigsäure hat nach der üblichen Aufarbeitung wie Destillation eine sehr hohe Reinheit. Man verwendet sie u. a. zur Herstellung von Estern und als Lösungsmittel.

Beispiel 1

Als Reaktor dient ein leeres Rohr aus V4A von 210 mm Durchmesser und 1 155 mm Höhe. Das Rohr geht in einen Kühler über, der aus einem Röhrenbündel besteht. Oberhalb vom Kühler befindet sich ein Trenngefäß für die Trennung Gas/Flüssigkeit. Das Gas entweicht über einen Totalkondensator. Die Flüssigkeit fließt in den Reaktor zurück. In diesen Flüssigkeitsstrom dosiert man 150 Normliter/h Luft (Nl/h) ein.

Man beschickt den Reaktor kontinuierlich mit 12 890 g/h sek.-Butylacetat (frisch), 6 540 Nl/h reinem Sauerstoff, 14 260 g/h eines auf 168 °C erwärmten Leichtsiederstromes (s. u.) und 65 ml einer Kontaktlösung von 50,7 g Kobaltacetat · 4H$_2$O in 1 l einer 60 %igen wäßrigen Essigsäure (12 g Co/l). Die Kobaltionen-Konzentration im Reaktionsgemisch beträgt 25 ppm. Man oxidiert das sek.-Butylacetat bei einer Temperatur von 170 °C und einem Druck von 13 bar. Bei diesen Temperaturbedingungen und der angegebenen Sauerstoffzugabe stellt sich ein Sauerstoff-Gehalt von ca. 4 Vol.-% in der Gasphase oberhalb der Flüssigkeit ein. Die Gesamtmenge der durch Korrosion in das Reaktionsmedium gelangenden Fremdmetallionen (Fe, Cr, Ni) ist kleiner als 5 ppm.

Entsprechend der Zugabe wird dem Reaktor kontinuierlich ein Flüssigkeitsstrom entzogen. Man erhält 3 334 g/h eines flüssigen Reaktoraustrages folgender Zusammensetzung :

50   % Essigsäure
27,4 % sek.-Butylacetat
7,8 % Methylethylketon und Ethylacetat
4,4 % Methylacetat
5,7 % Wasser
1,6 % Ameisensäure

3

0,9 % Propionsäure
2,2 % Sonstige (z.B. Acetaldehyd, Aceton, Ethanol)

Der flüssige Reaktoraustrag wird in einer nachgeschalteten sogenannten Leichtsiederkolonne in **Rohessigsäure folgender Zusammensetzung**

87,4 % Essigsäure
2,8 % Ameisensäure
1,6 % Propionsäure
8,2 % Wasser

und den bereits genannten Leichtsiederstrom zerlegt.
Der Leichtsiederstrom mit der Zusammensetzung

64,1 % sek.-Butylacetat
18,2 % Methylethylketon und Ethylacetat
10,3 % Methylacetat und
7,4 % Sonstige (einschließlich Wasser)

wird (s. o.) auf 168 °C erwärmt in den Reaktor zurückgeführt.
Bilanziert man den Prozeß unter dem Gesichtspunkt

1 Mol Buten (in Form von sek.-Butylacetat)⟶2 Mol Essigsäure

so erhält man bei Rückführung des nicht umgesetzten sek.-Butylacetats und der Zwischenprodukte folgende Werte für Selektivität, Raumzeitausbeute und Umsatz/Durchgang :

Selektivität : 74,8 %
Raum-Zeit-Ausbeute : 249 g Essigsäure/l Reaktionsvolumen · h
Umsatz/Durchgang : 47,5 %
Gesamtumsatz : ca. 100 %, bezogen auf sek.-Butylacetat

Der Kobalteinsatz für 1 kg erzeugte Essigsäure beträgt 79 mg.

## Beispiel 2

In der in Beispiel 1 beschriebenen Versuchsanlage beschickt man den Reaktor kontinuierlich mit 8 049 g/h sek.-Butylacetat (frisch), 20 650 Nl/h Luft, 12 232 g/h eines auf 173 °C erwärmten Leichtsiederstromes (s. u.) und 14 ml einer Kontaktlösung aus Kobaltacetat · $4H_2O$ in einer 60 %igen wäßrigen Essigsäure (12 g Co/l). Die Kobaltionen-Konzentration im Reaktionsgemisch beträgt 7 ppm. Wie im Beispiel 1 dosiert man 150 Nl/h Luft in den Kühler. Man oxidiert das sek.-Butylacetat bei einer Temperatur von 175 °C und einem Druck von 13,5 bar. Wie im Beispiel 1 stellt sich ein Sauerstoff-Gehalt von 1,5 Vol.-% in der Gasphase ein. Die Fremdmetallkonzentration ist ebenfalls kleiner als 5 ppm. Entsprechend der Zugabe entzieht man dem Reaktor kontinuierlich einen Flüssigkeitsstrom. Man erhält 24 139 g/h eines flüssigen Reaktoraustrages folgender Zusammensetzung :

42,6 % Essigsäure
37,5 % sek.-Butylacetat
7,7 % Methylethylketon und Ethylacetat
3,1 % Methylacetat
5,6 % Wasser
1,4 % Ameisensäure
0,5 % Propionsäure
1,4 % Sonstige (z.B. Acetaldehyd, Aceton, Ethanol)

In der Leichtsiederkolonne zerlegt man den Reaktoraustrag in Rohessigsäure folgender Zusammensetzung :

86,4 % Essigsäure
3,3 % Ameisensäure
1 % Propionsäure
9,3 % Wasser

und den bereits genannten Leichtsiederstrom.
Den Leichtsiederstrom mit der Zusammensetzung

4

74 % sek.-Butylacetat
15,2 % Methylethylketon und Ethylacetat
6,1 % Methylacetat
4,7 % Sonstige (einschl. Wasser)

führt man in den Reaktor zurück.

Man erhält unter Berücksichtigung der Rückführung des nicht umgesetzten sek.-Butylacetats und der Zwischenprodukte folgende Werte für Selektivität, Raumzeitausbeute und Umsatz/Durchgang:

| | |
|---|---|
| Selektivität: | 73,5 % |
| Raum-Zeit-Ausbeute: | 153 g Essigsäure/l · h |
| Umsatz/Durchgang: | 39,7 % |
| Gesamtumsatz: | ca. 100 %, bezogen auf Butylacetat |

Der Kobalteinsatz für 1 kg erzeugte Essigsäure beträgt 27 mg.

Beispiele 3 bis 8

Ein leeres Rohr aus V4A von 72 mm Durchmesser und 800 mm Länge dient als Blasensäulen-Reaktor und ist mit einem Rückflußkühler versehen. Man beschickt den Reaktor kontinuierlich mit 250 g/h sek.-Butylacetat (frisch), 150 Nl/h reinem Sauerstoff, 70 g/h Essigsäure, welche die in der Tabelle 1 angegebene Kobalt-Konzentration enthält. Man oxidiert das Reaktionsgemisch bei einer Temperatur von ca. 170 °C und einem Druck von ca. 11 bar. Am unteren Ende des Kühlers dosiert man 10 Nl/h Luft ein. Die Gesamtmenge der durch Korrosion in das Reaktionsmedium gelangenden Fremdmetallionen ist kleiner als 5 ppm. Entsprechend der Zugabe wird dem Reaktor kontinuierlich ein Flüssigkeitsstrom entzogen, von dem in einer nachgeschalteten Leichtsiederkolonne nicht umgesetztes sek.-Butylacetat und die bereits erwähnten anderen Leichtsieder abgetrennt und in den Oxidator zurückgefahren werden. Man erhält einen Produktstrom von 550 g/h, der analog zu Beispiel 1 zur Berechnung der Selektivität herangezogen wird. In der Tabelle 1 sind die für unterschiedliche Katalysator-Konzentrationen erhaltenen Selektivitäts-Werte aufgelistet.

TABELLE 1

| Beisp. Nr. | Kobalt-Konz. in Essigs. ppm | Kobalt-Konz. im Reaktor ppm | Selektivität zu | | $O_2$-Gehalt Vol.-% |
|---|---|---|---|---|---|
| | | | Essigs. | Ameisens. | |
| | | | Mol-% | | |
| 3 | 3 000 | 300 | 64,5 | 1,9 | 6 |
| 4 | 1 000 | 100 | 71,3 | 1,5 | 5 |
| 5 | 300 | 30 | 74,2 | 2,3 | 3 |
| 6 | 100 | 10 | 73,6 | 2,7 | 3 |
| 7 | 30 | 3 | 71,3 | 2,2 | 4 |
| 8 | 10 | 1 | 63,6 | 3,3 | 5 |

Eine Zusammenfassung der Werte erfolgt in der Abbildung 1.

(Siehe die Abbildung Seite 6)

Abb. 1

Beispiel 9

In der im Beispiel 3 beschriebenen Versuchsanlage beschickt man den Reaktor kontinuierlich mit 220 g/h sek.-Butylacetat (frisch), 140 Nl/h reinem Sauerstoff, 60 g/h Essigsäure, welche 300 ppm Kobaltionen enthält, so daß sich im Reaktor eine Kobalt-Konzentration von 30 ppm einstellt. Außerdem führt man dem Reaktor das Kopfprodukt der Leichtsiederdestillation zu. Man oxidiert das Reaktionsgemisch bei einer Temperatur von 164 °C und einem Druck von ca. 8,5 bar. Der Sauerstoff-Gehalt im Abgas beträgt ca. 4 Vol.-%. In den Kühler werden am untersten Ende ca. 10 Nl/h Luft eindosiert. Die Gesamtmenge der durch Korrosion in das Reaktionsmedium gelangenden Fremdmetallionen in kleiner als 3 ppm.

Analog zum Beispiel 3 erhält man einen Produktstrom von 470 g/h, der zur Berechnung der Selektivität herangezogen wird. Sie beträgt für Essigsäure 74,5 %, für Ameisensäure 1,9 %.

**Ansprüche**

1. Verfahren zur Herstellung von Essigsäure durch Oxidation von sek.-Butylacetat in flüssiger Phase bei Temperaturen von 150 bis 190 °C mit Sauerstoff oder sauerstoffhaltigen Gasen wie Luft, dadurch gekennzeichnet, daß man die Reaktion in Gegenwart von 1 bis 300 ppm Kobaltionen in der Reaktionslösung bei Drücken von 7 bis 30 bar durchführt und den Sauerstoffgehalt im gesamten Reaktionsraum einschließlich des Kühlers und der Rohrleitungen sowie im Abgas auf 1 bis 6 Vol.-%, vorzugsweise 3 bis 5 Vol.-% hält, wobei man zur Einhaltung dieses Sauerstoffgehaltes zusätzlich Sauerstoff in den Kühler und/oder in die Rohrleitung zwischen Reaktionsbehälter und Kühler eindosiert.

2. Verfahren nach Anspruch 1, dadurch gekennzeichnet, daß man die Reaktion in Gegenwart von 3 bis 100, vorzugsweise von 10 bis 50 ppm Kobaltionen durchführt.

3. Verfahren nach den Ansprüchen 1 und 2, dadurch gekennzeichnet, daß man die Reaktion bei einem Druck von 10 bis 20 bar durchführt.

4. Verfahren nach den Ansprüchen 1 bis 3, dadurch gekennzeichnet, daß man die Reaktionswärme mittels Siedekühlung abführt.

**Claims**

1. Process for the preparation of acetic acid by oxidation of sec.-butyl acetate in the liquid phase at temperatures from 150 to 190 °C with oxygen or oxygen-containing gases, such as air, characterised in that the reaction is carried out in the presence of 1 to 300 ppm of cobalt ions in the reaction solution under pressures from 7 to 30 bar and the oxygen content in the entire reaction space, including the condenser and the pipes as well as the exit gas, is maintained at 1 to 6 % by volume, preferably 3 to 5 % by volume, additional oxygen being metered into the condenser and/or into the pipe between the reaction vessel and the condenser, in order to maintain this oxygen content.

2. Process according to Claim 1, characterised in that the reaction is carried out in the presence of 3 to 100, preferably 10 to 50, ppm of cobalt ions.

3. Process according to Claims 1 and 2, characterised in that the reaction is carried out under a pressure from 10 to 20 bar.

4. Process according to Claims 1 to 3, characterised in that the heat of reaction is removed by means of evaporative cooling.

**Revendications**

1. Procédé de préparation d'acide acétique par oxydation d'acétate de butyle secondaire en phase liquide à des températures de 150 à 190 °C, avec de l'oxygène ou des gaz oxygénés tels que l'air, caractérisé par le fait qu'on effectue la réaction en présence de 1 à 300 ppm d'ions cobalt dans la solution de réaction, à des pressions de 7 à 30 bar et qu'on maintient la teneur en oxygène dans l'ensemble de la chambre de réaction, y compris le réfrigérant et les tubulures, ainsi que dans le gaz d'échappement, à une valeur de 1 à 6 % en volume, de préférence de 3 à 5 % en volume, tandis que, pour le maintien de cette teneur en oxygène, on introduit en plus de l'oxygène dans le réfrigérant et/ou dans la tubulure entre le récipient de réaction et le réfrigérant.

2. Procédé selon la revendication 1, caractérisé par le fait qu'on effectue la réaction en présence de 3 à 100, de préférence de 10 à 50 ppm d'ions cobalt.

3. Procédé selon les revendications 1 et 2, caractérisé par le fait qu'on effectue la réaction à une pression de 10 à 20 bar.

4. Procédé selon les revendications 1 à 3, caractérisé par le fait qu'on évacue la chaleur réactionnelle à l'aide d'un refroidissement par vaporisation.